# EUROPÄISCHE PATENTSCHRIFT

(11) **EP 0 479 024 B1**
(45) Veröffentlichungstag und Bekanntmachung des Hinweises auf die Patenterteilung: **22.12.1993**
(21) Anmeldenummer: 91115665.1
(22) Anmeldetag: 16.09.1991
(51) Int. Cl.: H05G 1/26, H05G 1/44, H05G 1/46, A61B 6/00

(54) **Röntgendiagnostikanlage**
X-ray diagnostic apparatus
Installation de radiodiagnostic

(30) Priorität: 29.09.1990 DE 4030906
(43) Veröffentlichungstag der Anmeldung: 08.04.1992
(73) Patentinhaber: SIEMENS AKTIENGESELLSCHAFT, 80333 München (DE)
(72) Erfinder: Aichinger, Horst, Dr., W-8150 Fürth (DE); Joite-Barfuss, Sigrid, W-8520 Erlangen (DE); Köhler, Karlheinz, W-8522 Herzogenaurach (DE)

(56) Entgegenhaltungen:
- EP-A- 0 325 120
- EP-A- 0 346 530
- US-A- 4 669 105
- ELECTRO MEDICA (SIEMENS), Band 58, Nr. 2, August 1990, Erlangen, DE;Seiten 61-66, H.AICHINGER u.a.: "Die Beleuchtungsautomatik in der Mammographie"

## Beschreibung

Die Erfindung betrifft eine Röntgendiagnostikanlage für Mammographieaufnahmen mit einem Röntgenstrahler, einem diesem gegenüberliegenden Lagerungstisch und einem Rechner für die Ermittlung der mittleren Parenchymdosis.

Zur Abschätzung des Strahlenrisikos ist die Kenntnis der Strahlenexposition der Patientin bei der Anfertigung von Röntgenaufnahmen von Interesse. Die hierfür relevante Dosisgröße ist die mittlere Parenchymdosis. Sie kann durch Messung der Eintrittsdosis ermittelt werden. Eine solche Messung ist jedoch bei der Anfertigung von Röntgenaufnahmen mit einer Patientin in der Regel nicht möglich, da hierzu ein schattengebender Detektor vor dem Aufnahmeobjekt angeordnet werden müßte.

In der EP-A-0 325 120 ist zur Bestimmung der mittleren Parenchymdosis vorgeschlagen, einen Rechner zu verwenden, dem den jeweiligen Aufnahmewerten entsprechende elektrische Signale zugeführt werden und der daraus die mittlere Parenchymdosis nach einer vorgegebenen Formel berechnet. Eine echte Messung erfolgt bei diesem Stand der Technik demgemäß nicht.

Der Erfindung liegt die Aufgabe zugrunde, eine Röntgendiagnostikanlage der eingangs genannten Art so auszubilden, daß die mittlere Parenchymdosis durch eine echte Messung ermittelt wird.

Diese Aufgabe ist erfindungsgemäß dadurch gelöst, daß dem Rechner das Ausgangssignal eines auf dem Lagerungstisch neben dem Meßfeld angeordneten Strahlendetektors zugeführt wird, wobei der Rechner entsprechend der Abhängigkeit der mittleren Parenchymdosis von der vom Strahlendetektor gemessenen Dosis programmiert ist. Bei der erfindungsgemäßen Röntgendiagnostikanlage wird die Dosis neben dem Aufnahmeobjekt am Lagerungstisch gemessen. Aufgrund dieser Dosis und der geometrischen Lage des Strahlendetektors kann die Eintrittsdosis und damit die dieser entsprechende mittlere Parenchymdosis berechnet werden.

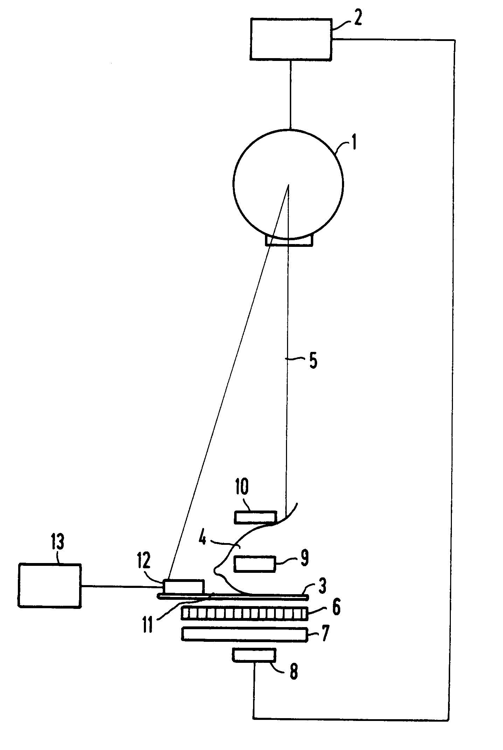

Die Erfindung ist nachfolgend anhand eines in der Zeichnung dargestellten Ausführungsbeispieles näher erläutert.

In der Zeichnung ist ein Röntgenstrahler 1 dargestellt, der von einem Röntgengenerator 2 gespeist wird und eine auf einem Lagerungstisch 3 liegende Mamma 4 mit einem Röntgenstrahlenbündel 5 durchstrahlt. In Strahlenrichtung hinter dem Lagerungstisch 3 ist ein Sekundärstrahlenraster 6 zur Unterdrückung der Streustrahlung, eine Filmkassette 7 mit einem Röntgenfilm und der Strahlendetektor 8 eines Röntgenbelichtungsautomaten angeordnet, welcher am Röntgengenerator 2 zur Bestimmung der Einschaltzeit angeschlossen ist.

Die mittlere Parenchymdosis ist die im Bereich 9 der Mamma 4 wirkende Dosis. Sie könnte durch einen Strahlendetektor an der Stelle 10 als Eintrittsdosis gemessen werden. Bei einer Mammaaufnahme stört jedoch ein solcher Strahlendetektor an der Stelle 10.

Zur Bestimmung der mittleren Parenchymdosis ist auf dem Lagerungstisch 3 neben dem Meßfeld 11, in dem die Mamma 4 liegt, ein Strahlendetektor 12 angeordnet, der an einem Rechner 13 angeschlossen ist. Der Strahlendetektor 12 mißt die Dosis an seinem Ort und führt dem Rechner 13 ein entsprechendes Signal zu. Der Rechner 13 ist entsprechend der Abhängigkeit der mittleren Parenchymdosis im Bereich 9 von der vom Strahlendetektor 12 gemessenen Dosis programmiert und berechnet demgemäß die mittlere Parenchymdosis.

## Patentansprüche

1. Röntgendiagnostikanlage für Mammographieaufnahmen mit einem Röntgenstrahler (1), einem diesem gegenüberliegenden Lagerungstisch (3) und einem Rechner (13) für die Ermittlung der mittleren Parenchymdosis, **dadurch gekennzeichnet,** daß dem Rechner (13) das Ausgangssignal eines auf dem Lagerungstisch (3) neben dem Meßfeld (11) angeordneten Strahlendetektors (12) zugeführt wird, wobei der Rechner (13) entsprechend der Abhängigkeit der mittleren Parenchymdosis von der vom Strahlendetektor (12) gemessenen Dosis programmiert ist.

## Claims

1. X-ray diagnostic system for mammography exposures having an X-ray source (1), a positioning table (3) lying opposite the latter and a computer (13) for the determination of the average parenchyma dose, characterised in that the output signal of a radiation detector (12), which is arranged on the positioning table (3) next to the measurement field (11), is supplied to the computer (13), the latter being programmed in accordance with the dependence of the average parenchyma dose upon the dose measured by the radiation detector (12).

## Revendications

1. Installation de radiodiagnostic pour des radiographies mammaires, comportant un émetteur radiologique (1), une table de support (3) située en vis-à-vis de cet émetteur et un ordinateur (13) servant à déterminer la dose moyenne de parenchyme, caractérisée par le fait que le signal de sortie d'un détecteur de rayonnement (12) disposé sur la table de support (13) à côté du champ de mesure (11) est envoyé à l'ordinateur (13), ce dernier étant programmé sur la base de la variation de la dose moyenne de parenchyme en fonction de la dose mesurée par le détecteur de rayonnement (12).
